# EUROPÄISCHE PATENTSCHRIFT

(11) **EP 1 361 279 B1**
(45) Veröffentlichungstag und Bekanntmachung des Hinweises auf die Patenterteilung: **05.07.2006**
(21) Anmeldenummer: 03010224.8
(22) Anmeldetag: 07.05.2003
(51) Int. Cl.: C12P 13/04

(54) **Verfahren zur enzymatischen Herstellung enantiomerenangereicherter beta-Aminosäuren**
Method for enzymatic production of enantiomerically enriched beta amino acids
Procédé pour la préparation d'amino-acides beta enrichis en énantiomères

(30) Priorität: 08.05.2002 DE 10220739
(43) Veröffentlichungstag der Anmeldung: 12.11.2003
(73) Patentinhaber: Degussa AG, 40474 Düsseldorf (DE)
(72) Erfinder: Gröger, Harald, Dr., 63450 Hanau (DE); Werner, Helge, 63486 Bruchköbel (DE)

(56) Entgegenhaltungen:
- WO-A-01/16090
- US-A- 5 552 317
- FAULCONBRIDGE S J ET AL: "Preparation of enantiomerically enriched aromatic beta-amino acids via enzymatic resolution" TETRAHEDRON LETTERS, ELSEVIER SCIENCE PUBLISHERS, AMSTERDAM, NL, Bd. 41, Nr. 15, April 2000 (2000-04), Seiten 2679-2681, XP004194578 ISSN: 0040-4039
- DATABASE CROSSFIRE / BEILSTEIN [Online] Beilstein Registry Number 2939300, 2. Juni 1992 (1992-06-02) "3-Amino-3-phenyl-propionsäure-propylester " XP002248822

## Beschreibung

Die vorliegende Erfindung bezieht sich auf ein Verfahren zur Herstellung von enantiomerenangereicherten β-Aminosäuren.

Optisch aktive β-Aminocarbonsäuren treten in Naturstoffen wie Alkaloiden und Antibiotika auf, und deren Isolierung gewinnt zunehmend an Interesse, nicht zuletzt wegen deren steigender Bedeutung als essentielle Zwischenprodukte bei der Herstellung von Arzneimitteln (siehe u.a.: E. Juaristi, H. Lopez-Ruiz, *Curr. Med. Chem.* 1999, *6*, 983-1004). Sowohl die freie Form optisch aktiver β-Aminocarbonsäuren als auch deren Derivate zeigen interessante pharmakologische Effekte und können auch bei der Synthese modifizierter Peptide eingesetzt werden.

Als Herstellungsmethoden für β-Aminocarbonsäuren haben sich bislang die klassische Racematspaltung über diastereomere Salze (vorgeschlagene Route in: H. Boesch et al., Org. Proc. Res. Developm. 2001, 5, 23-27) und insbesondere die diastereoselektive Addition von Lithium-Phenylethylamid (A. F. Abdel-Magid, J. H. Cohen, C. A. Maryanoff, *Curr. Med*. *Chem*. 1999, *6*, 955-970) etabliert. Letztere Methode gilt als intensiv erforscht und wird trotz zahlreicher dabei auftretender Nachteile bevorzugt angewandt. Zum einen werden stöchiometrische Mengen eines chiralen Reagenzes benötigt, was im Vergleich zu katalytischen asymmetrischen Methoden einen großen Nachteil darstellt. Außerdem werden teure und zudem gefährliche Hilfsstoffe wie beispielsweise *n*-Butyllithium zur Aktivierung des stöchiometrischen Reagenz durch Deprotonierung benötigt. Für eine genügende Stereoselektivität ist zudem die Durchführung der Reaktion bei niedrigen Temperaturen von ca. -70°C wichtig, was einen hohen Anspruch an das Reaktormaterial, zusätzliche Kosten und einen hohen Energieverbrauch bedeutet.

Die Herstellung von optisch aktiven β-Aminocarbonsäuren auf biokatalytischem Wege spielt zwar gegenwärtig nur eine untergeordnete Rolle, ist aber insbesondere aufgrund der ökonomischen und ökologischen Vorteile biokatalytischer Reaktionen wünschenswert. Es entfällt der Einsatz stöchiometrischer Mengen eines chiralen Reagenzes und stattdessen werden geringe, katalytische Mengen von Enzymen eingesetzt, die natürliche und umweltfreundliche Katalysatoren darstellen. Diese im wässrigen Medium effizient eingesetzten Biokatalysatoren weisen neben ihren katalytischen Eigenschaften und ihrer hohen Wirksamkeit zudem, im Gegensatz zu einer Vielzahl von synthetischen metallhaltigen Katalysatoren, den Vorteil auf, dass auf die Verwendung metallhaltiger, insbesondere schwermetallhaltiger und somit toxischer Einsatzstoffe verzichtet werden kann.

Im Stand der Technik wurde z.B. bereits mehrfach über die enantioselektive *N*-Acylierung von β-Aminocarbonsäuren berichtet.

So beschreiben L.T. Kanerva et al. in Tetrahedron:
Asymmetry, Vol. 7, No. 6, S. 1707-1716, 1996 die enantioselektive N-Acylierung von Ethylestern verschiedener alicylischer β-Aminocarbonsäuren mit 2,2,2-Trifluorethylester in organischen Lösungsmitteln und Lipase SP 526 aus *Candida antarctica* oder Lipase PS aus *Pseudomonas cepacia* als Biokatalysator.
V.M. Sänchez et al. untersuchten die biokatalytische Racematspaltung von (±)-Ethyl-3-aminobutyrat (Tetrahedron: Asymmetry, Vol. 8, No. 1, S. 37-40, 1997) mit Lipase aus *Candida antarctica* über die Herstellung des *N*-acetylierten β-Aminocarbonsäureesters.

In EP-A-8 890 649 ist ein Verfahren zur Herstellung von optisch aktiven Aminosäureestern aus racemischen Aminosäureestern durch enantioselektive Acylierung mit einem Carbonsäureester in Gegenwart einer Hydrolase, ausgewählt aus der Gruppe Amidase, Protease, Esterase und Lipase, und nachfolgende Isolierung des nicht umgesetzten Enantiomers des Aminosäureesters offenbart.

WO-A-98/50575 betrifft ein Verfahren zur Gewinnung einer chiralen β-Aminocarbonsäure oder ihrer entsprechenden Ester durch Inberührungbringen einer racemischen β-Aminocarbonsäure, eines Acyldonors und Penicilin G Acylase unter Bedingungen, um ein Enantiomer der racemischen β-Aminocarbonsäure stereoselektiv zu acylieren, wobei das andere Enantiomer im wesentlichen nicht umgesetzt wird, und man so eine chirale β-Aminocarbonsäure erhält.
Auch die umgekehrte Reaktionsfolge ist untersucht worden (V. A. Soloshonok, V. K. Svedas, V. P. Kukhar, A. G. Kirilenko, A. V. Rybakova, V. A. Solodenko, N. A. Fokina, O. V. Kogut, I. Y. Galaev, E. V. Kozlova, I. P. Shishkina, S. V. Galushko, Synlett 1993, 339-341; V. Soloshonok, A. G. Kirilenko, N. A. Fokina, I. P. Shishkina, S. V. Galushko, V. P. Kukhar, V. K. Svedas, E. V. Kozlova, Tetrahedron: Asymmetry 1994, 5, 1119-1126; V. Soloshonok, N. A. Fokina, A. V. Rybakova, I. P. Shishkina, S. V. Galushko, A. E. Sochorinsky, V. P. Kukhar, M. V. Savchenko, V. K. Svedas, Tetrahedron: Asymmetry 1995, 6, 1601-1610; G. Cardillo, A. Tolomelli, C. Tomasini, Eur. J. Org. Chem. 1999, 155-161). Nachteilig bei diesem Verfahren ist die schwierige Aufarbeitung des Produktgemisches nach der enantioselektiven Hydrolyse. Nach Abtrennung der freien β-Aminocarbonsäure erhält man ein Gemisch aus Phenylessigsäure und *N*-Phenylacetyl-β-Aminocarbonsäure, das schwierig aufzutrennen ist.

Zur Gewinnung von enantiomerenangereicherten Carbonsäuren ist seit längerer Zeit schon deren Umsetzung mit Lipasen bekannt. In der US5518903 ist dieses Prinzip auf N-geschützte β-Aminosäureester mit allerdings wechselndem Erfolg übertragen worden. Während einzig der entsprechende Benzylester von racemischer N-Butoxycarbonyl-β-aminobuttersäure hoch enantioselektiv mittels einer Lipase gespalten werden konnte, lieferten die restlichen eingesetzten Methylester bzw. n-Butylester lediglich ee-Werte im Bereich von 70%. Es ist dabei festzustellen, dass offensichtlich ein Übergang von einem entsprechenden Methylester zu einem n-Butylester mit einer Verschlechterung des ee-Wertes der hergestellten Säure einhergeht. So ergibt die Esterhydrolyse ausgehend vom n-Butylester der N-Boc-β-aminobuttersäure mit dem Enzym Lipase von der Firma Asahi nach 8 Tagen in 37% Ausbeute einen ee-Wert der entsprechenden Säure von 45%ee. Mit der Lipase PS von Amano erhält man bei der gleichen Reaktion immerhin innerhalb von 7 Tagen mit einer Ausbeute von 41% eine zu 61%ee angereicherte Verbindung. Im Vergleich dazu liefert der entsprechende Methylester 70%ee.

Den kürzlich von Faulconbridge et al. veröffentlichten Ergebnissen ist zu entnehmen, dass die Esterhydrolyse von aromatischen β-Aminosäureethylestern bei pH 8 mit der Lipase PS von Amano in annehmbaren Ausbeuten und sehr guten Enantiomerenüberschüssen erfolgt (Tetrahedron Letters 2000, 41, 2679-81). Das Produkt wird mit einer Enantiomerenreinheit von bis zu 99% erhalten, allerdings ist die Synthese, die ausschließlich in Suspension durchgeführt wurde, mit einigen Nachteilen verbunden. Zum einen hat sich gezeigt, dass zwar die Kristallisation unter diesen Bedingungen selektiv ist, allerdings die Reaktion an sich, wie im Vergleichsbeispiel 1 dokumentiert, zu niedrigeren ee-Werten von 85.1% ee führt. Insgesamt bedeutet dies einerseits einen Ausbeuteverlust aufgrund der Bildung des ungewünschten Enantiomers, zum anderen führt dies dazu, dass der ee-Wert in Abhängigkeit von leichten Prozessschwankungen im technischen Maßstab aufgrund veränderter Kristallisationsbedingungen leicht auch unter 99% ee bzw. sogar unter 98% ee fallen kann. Ein möglichst hoher ee-Wert von >98% ee, insb. >99% ee, ist aber für Pharmaanwendungen eine Voraussetzung. Darüber hinaus wäre auch die Durchführung in homogenem Medium wünschenswert (keine Suspension!), um eine gute Enzymabtrennung *via* Ultrafiltration gewährleisten zu können. Optimalerweise sollte in diesem Schritt ebenfalls ein hoher ee-Wert anfallen, was mit dem bisherigen Literaturverfahren nicht realisiert werden kann. Aufgabe der vorliegenden Erfindung war daher die Angabe eines weiteren Verfahrens zur enzymatischen Herstellung von β-Aminosäuren. Insbesondere sollte dieses Verfahren unter ökonomischen wie ökologischen Gesichtspunkten vorteilhaft in einem technischen Maßstab einsetzbar sein, d.h. in Bezug auf die Umweltverträglichkeit, den Arbeitsschutz und die Robustheit des Prozesses sowie die Raum/Zeit-Ausbeute und Selektivität besonders hervorstechen.

Diese und weitere nicht näher genannte sich jedoch aus dem Stand der Technik in naheliegenderweise ergebende Aufgaben werden gelöst durch ein Verfahren mit den Merkmalen des gegenständlichen Anspruchs 1. Abhängige Ansprüche 2 bis 8 beziehen sich auf bevorzugte Ausführungsformen der vorliegenden Erfindung.

Dadurch, dass man ein Verfahren zur Herstellung enantiomerenangereicherter N-ungeschützter β-Aminosäuren durch enzymatische Hydrolyse eines Enantiomerengemisches von N-ungeschützten β-Aminosäurealkylestern oder β-Aminosäurearylestern mit einer Lipase unter der Maßgabe durchführt, dass kein entsprechender Methyl- oder Ethylester eingesetzt wird, gelangt man sehr überraschend, dafür aber in nicht minder vorteilhafter Art und Weise zur Lösung der gestellten Aufgabe. Bisher wurden für die betrachtete Reaktion lediglich Methyl- oder Ethylester der N-ungeschützten β-Aminosäuren eingesetzt, die jedoch die oben schon erwähnten Nachteile mit sich bringen. Offensichtlich ergibt sich ein sowohl von der Raumzeitausbeute als auch von der Selektivität her gesehen besseres Resultat, wenn raumerfüllendere Estergruppen mit z.B. (C₃-C₈)-Alkylresten zur enzymatischen Hydrolyse herangezogen werden. Dies ist einerseits im Hinblick auf die oben besprochene US 5518903 als Überraschung zu werten, andererseits ist dieser Trend unerwartet, da bei einer schnelleren Reaktion die Wahrscheinlichkeit einer Enantiodifferenzierung gemeinhin abnimmt. Dieser logische Zusammenhang sei beispielhaft verdeutlicht, wenn man die im Allgemeinen niedrigeren Enantioselektivitäten bei höheren Reaktionstemperaturen - bei denen die Reaktion dafür schneller abläuft - betrachtet.

Im Prinzip ist der Fachmann frei in der Wahl der entsprechenden Estergruppe. Er wird sich bei seiner Auswahl an ökonomischen und reaktionstechnischen Gesichtspunkten orientieren. Günstige Alkohole zur Bildung des Esters sind insbesondere solche, die leicht aus dem Reaktionsgemisch entfernt werden können. Dies sind Alkohole wie Alkylalkohole oder Arylalkohole, ggf. niedrig siedendes Phenol oder Benzylalkohol. Die mit diesen Alkoholen erhältlichen β-Aminosäurealkylester oder β-Aminosäurearylester werden daher bevorzugt in die Hydrolyse eingesetzt. Ganz besonders bevorzugt ist der Einsatz von ensprechenden n-Propyl-, i-Propyl, n-Butyl, sec-Butyl-, i-Butyl oder tert.-Butylestern der β-Aminosäuren.

Die Wahl der Reaktionsparameter sind dem Fachmann ebenfalls freigestellt. Er wird sie anhand von Routineexperimenten für den Einzelfall gesondert ermitteln. Auf jeden Fall eignet sich für das gegenständliche enzymatische Verfahren ein pH-Wertebereich zwischen 4 und 10, vorzugsweise zwischen 6 und 9 und mehr bevorzugt zwischen 7 und 8,5 liegt. Um pH 8 hat sich die Lipase PS der Firma Amano als besonders geeignet herausgestellt.

In Hinblick auf die Temperatur liegen prinzipiell die gleichen Vorraussetzungen vor, wie für den pH-Wert. Auch hier kann eine möglichst optimale Temperatur für den Einzelfall ermittelt werden, je nachdem welches Enzym bei welcher Temperatur am optimalsten arbeitet. Für Enzyme aus thermophilen Organismen sind hohe Temperaturen bis 100°C möglich. Andere wiederum arbeiten optimal erst bei <0°C bis -15°C, ggf. in einer Eismatrix. Bevorzugt sollte die eingestellte Temperatur während der Reaktion im Bereich zwischen 15 und 40°C und mehr bevorzugt zwischen 20 und 30°C liegen.

Die Wahl des einzusetzenden Lipasen obliegt dem Fachmann. Aus Enzyme Catalysis in Organic Synthesis, Ed.: K. Drauz, H. Waldmann, VCH, 1995, S. 165 und der dort zitierten Literatur sind viele geeignete Enzyme auswählbar. Vorzugsweise wird für die Esterhydrolyse die Lipase PS von Amano aus *Pseudomonas cepacia* eingesetzt.
Für die Anwendung kann das betrachtete Polypeptid in freier Form als homogen aufgereinigte Verbindung oder als rekombinant hergestelltes Enzym verwendet werden. Weiterhin kann das Polypeptid auch als Bestandteil eines intakten Gastorganismus eingesetzt werden oder in Verbindung mit der aufgeschlossenen und beliebig hoch aufgereinigten Zellmasse des Wirtsorganismus.
Möglich ist ebenfalls die Verwendung der Enzyme in immobilisierter Form (Sharma B. P.; Bailey L. F. und Messing R. A. (1982), Immobilisierte Biomaterialien-Techniken und Anwendungen, Angew. Chem. 94, 836-852). Vorteilhafterweise erfolgt die Immobilisierung durch Lyophilisation (Paradkar, V. M.; Dordick, J. S. (1994), Aqueous-Like Activity of α-Chymotrypsin Dissolved in Nearly Anhydrous Organic Solvents, J. Am. Chem. Soc. 116, 5009-5010; Mori, T.; Okahata, Y. (1997), A variety of lipicoated glycoside hydrolases as effective glycosyl transfer catalysts in homogeneous organic solvents, Tetrahedron Lett. 38, 1971-1974; Otamiri, M.; Adlercreutz, P.; Matthiasson, B. (1992), Complex formation between chymotrypsin and ethyl cellulose as a means to solubilize the enzyme in active form in toluene, Biocatalysis 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-mono-cetylether) (Kamiya, N.; Okazaki, S.-Y.; Goto, M. (1997), Surfactant-horseradish peroxidase complex catalytically active in anhydrous benzene, Biotechnol. Tech. 11, 375-378).
Äußerst bevorzugt ist die Immobilisierung an Eupergit® , insbesondere Eupergit C® und Eupergit 250L® (Röhm) (als Übersicht, siehe: E. Katchalski-Katzir, D. M. Kraemer, *J*. *Mol*. *Catal*. *B: Enzym.* **2000**, *10*, 157). Gleichfalls bevorzugt ist die Immobilisierung an Ni-NTA in Kombination mit dem durch Anhängen eines His-Tags (Hexa-Histidin) veränderten Polypeptid (Petty, K.J. (1996), Metal-chelate affinity chromatography In: Ausubel, F.M. et al. eds. *Current Protocols in Molecular Biology,* Vol. 2, New York: John Wiley and Sons).
Die Verwendung als CLECs ist ebenfalls denkbar (St. Clair, N.; Wang, Y.-F.; Margolin, A. L. (2000), Cofactor-bound cross-linked enzyme crystals (CLEC) of alcohol dehydrogenase, Angew. Chem. Int. Ed. 39, 380-383).
Durch diese Maßnahmen kann es gelingen, aus Polypeptiden, welche durch organische Solventien instabil werden, solche zu generieren, die in Gemischen von wässrigen und organischen Lösungsmitteln bzw. ganz in Organik arbeiten können.

Die gegenständliche Reaktion kann in jedwedem dafür vorgesehenem Reaktionsgefäß durchgeführt werden. Dies sind im einzelnen normale Batchreaktoren, Schlaufenreaktoren oder ein Enzym-Membran-Reaktor (Bommarius, A. S.; Drauz, K.; Groeger, U.; Wandrey, C.; Membrane Bioreactors for the Production of Enantiomerically Pure α-Amino Acids, in: Chirality in Industry (Hrsg.: Collins, A. N.; Sheldrake, G. N.; Crosby, J.) 1992, John Wiley & Sons, S. 371-397).

Die bei der erfindungsgemäßen Reaktion einzusetzenden Ester zeigen mitunter eine schlechte Löslichkeit im wässrigen Reaktionsmedium. In diesen Fällen kann es abhängig von der Lösungsmitteltoleranz der eingesetzten Enzyme vorteilhaft sein, zur Erlangung einer homogenen Reaktionsphase wasserlösliche organische Lösungsmittel dem Reaktionsgemisch zuzugeben. Als solche wasserlöslichen organischen Lösungsmittel kommen u.a. im einzelnen in Frage: Aceton, DMF, Ethanol, Methanol.
Die Reaktion gelingt aber auch bei höheren Substratkonzentrationen unter Ausbildung einer Suspension.

Im Falle des Einsatzes von Enzymen, die ggf. auf wasserunlöslichen Trägermaterialien bzw. Begleitstoffen oder Stabilisatoren adsorbiert vorliegen, hat es sich als vorteilhaft erwiesen, den unlöslichen Träger bzw. Begleitstoff oder Stabilisator vor Einsatz des Enzyms in der Reaktion abzutrennen, damit eine Kontamination des auszufällenden Produktes mit dem unlöslichen Trägermaterial des eingesetzten Enzyms unterbleibt, sofern die Trennung von Enzym und Träger einfach möglich ist. Beispielsweise ist die vorteilhaft anzuwendende Lipase PS der Firma Amano auf Silicaträgern adsorbiert. Hier sollte daher vor der Zugabe der Reaktanden zum Reaktionsmedium eine Filtration der wässrigen Enzymlösung erfolgen, um die Kieselsäuren aus dem Reaktionsystem zu entfernen. Die Aktivität oder Prozessstabilität des Enzyms beeinflusst diese Vorgehensweise nicht negativ.

Unter "N-ungeschützt" wird im Rahmen der Erfindung die Tatsache verstanden, dass das β-Stickstoffatom der Säure nicht durch eine unter den Reaktionsbedingungen stabile N-Schutzgruppe blockiert ist. Als solche sind insbesondere die gängigen Schutzgruppen wie Z-, Boc-, Fmoc-, Eoc-, Moc-, Acetyl- etc. zu sehen.

Als (C₃-C₈)-Alkyl sind anzusehen n-Propyl, Isopropyl, n-Butyl, Isobutyl, sec-Butyl, tert-Butyl, Pentyl, Hexyl, Heptyl oder Octyl samt aller Bindungsisomeren. Diese können einfach oder mehrfach mit (C₁-C₈)-Alkoxy, (C₁-C₈)-Haloalkyl, OH, Halogen, NH₂, NO₂, SH, S-(C₁-C₈)-Alkyl substituiert sein.

Unter dem Begriff enantiomerenangereichert wird im Rahmen der Erfindung der Anteil eines Enantiomers im Gemisch mit seiner optischen Antipode in einem Bereich von >50 % und <100 % verstanden.

Die dargestellten Strukturen beziehen sich auf alle möglichen Diastereomere und Enantiomere und deren Gemische, die möglich sind.

### Experimentelle Beispiele:

### Beispiel 1 (= Vergleichsbeispiel):

Es werden 9.2 mmol der racemischen Verbindung *rac*-3-Amino-3-phenylpropionsäureethylester (1.79 g) in 50 mL Wasser aufgenommen und mittels automatischer pH-Statierung durch Zugabe von 1 M Natronlauge (bezug von Fa. Merck) die Lösung auf einen pH-Wert von pH 8.2 eingestellt. Um den Ester restlos in Lösung zu bringen, werden noch 3 mL Aceton zum Lösen hinzugefügt. Bei Erreichen einer Reaktionstemperatur von 20 °C werden 200 mg Amano Lipase PS (*Pseudomonas cepacia;* bezug von Fa. Amano Enzymes, Inc.) zugegeben um die Reaktion zu starten. Nach einer Reaktionszeit von 3 und 6 Stunden wird die Umsatzrate sowie nach 6 Stunden die Enantioselektivität des gebildeten (*S*)-3-Amino-3-phenylpropionsäure bestimmt. Dabei wird ein Umsatz von 18.5% nach 3 Stunden bzw. 37.8% nach 6 Stunden und eine Enantioselektivität von 85.1% ee (nach 6 Stunden) ermittelt. Die Umsatz- und Enantioselektivitätsbestimmung erfolgte *via* HPLC.

### Beispiel 2:

Es werden 9.2 mmol der racemischen Verbindung *rac*-3-Amino-3-phenylpropionsäure-*n*-propylester (1.91 g) in 50 mL Wasser aufgenommen und mittels automatischer pH-Statierung durch Zugabe von 1 M Natronlauge (bezug von Fa. Merck) die Lösung auf einen pH-Wert von pH 8.2 eingestellt. Um den Ester restlos in Lösung zu bringen, werden noch 3 mL Aceton zum Lösen hinzugefügt. Bei Erreichen einer Reaktionstemperatur von 20 °C werden 200 mg Amano Lipase PS (*Pseudomonas cepacia;* bezug von Fa. Amano Enzymes, Inc.) zugegeben um die Reaktion zu starten. Nach einer Reaktionszeit von 1 Stunde wird die Umsatzrate sowie nach 3 Stunden die Enantioselektivität des gebildeten (*S*)-3-Amino-3-phenylpropionsäure bestimmt. Dabei wird nach einer Stunde ein Umsatz von 48.7 % und eine Enantioselektivität von 96.4% ee (nach 3 Stunden) ermittelt. Die Umsatz- und Enantioselektivitätsbestimmung erfolgte *via* HPLC.

### Beispiel 3:

Es werden 8.63 mmol der racemischen Verbindung *rac*-3-Amino-3-phenylpropionsäure-*n*-butylester (1.91 g) in 50 mL Wasser aufgenommen und mittels automatischer pH-Statierung durch Zugabe von 1 M Natronlauge (bezug von Fa. Merck) die Lösung auf einen pH-Wert von pH 8.2 eingestellt. Um den Ester restlos in Lösung zu bringen, werden noch 3 mL Aceton zum Lösen hinzugefügt. Bei Erreichen einer Reaktionstemperatur von 20 °C werden 200 mg Amano Lipase PS (*Pseudomonas cepacia;* bezug von Fa. Amano Enzymes, Inc.) zugegeben um die Reaktion zu starten. Nach einer Reaktionszeit von 3 Stunden wird sowohl die Umsatzrate als auch die Enantioselektivität des gebildeten (*S*)-3-Amino-3-phenylpropionsäure bestimmt. Dabei wird ein Umsatz von 45.2 % und eine Enantioselektivität von 96.8% ee ermittelt. Die Umsatz- und Enantioselektivitätsbestimmung erfolgte *via* HPLC.

### Beispiel 4:

Es werden 81 mL Wasser vorgelegt und dazu 1.45 g Amano Lipase PS (*Pseudomonas cepacia;* Bezug durch Fa. Amano Enzymes, Inc.) zugegeben. Anschließend filtriert man den ungelösten Feststoff ab. Die als Filtrat resultierende wäßrige Enzymlösung wird mit 81 mL Methyl-*tert*-butylether (MTBE) als organische Solvenskomponente versetzt. Das entstandene Zweiphasensystem wird mittels automatischer pH-Statierung durch Zugabe von 1 M Natronlauge (Bezug durch Fa. Merck) auf pH 8.2 eingestellt. Bei Erreichen einer Temperatur von 20 °C werden dann 188.2 mmol der racemischen Verbindung *rac*-3-Amino-3-phenylpropionsäure-*n*-propylester (39.0 g) hinzugegeben, und die Reaktion gestartet. Die Reaktionszeit beträgt 15 Stunden, wobei ein weißer Niederschlag, bestehend aus dem gewünschten Produkt (*S*)-3-Amino-3-phenylpropionsäure, anfällt. Nach einer Reaktionszeit von 15 Stunden werden 160 mL Aceton zur Komplettierung der Fällung zugefügt, ca. 45 Minuten nachgerührt und der Feststoff abfiltriert. Der Feststoff wird mehrmals mit wenig Aceton gewaschen und anschließend im Vakuum getrocknet. Es werden 12.91 g der gewünschten (*S*)-3-Amino-3-phenylpropionsäure erhalten, entsprechend einer Ausbeute von 41.6%. Die Enantioselektivität für das Produkt liegt bei 99.6% ee. Die Enantioselektivitätsbestimmung erfolgte *via* HPLC. Für die chemische Reinheit wurde 98.8% ermittelt (ermittelt *via* Titration). Die Struktur des Produkts wurde zusätzlich *via* NMR-Spektroskopie bestätigt.

## Patentansprüche

1. Verfahren zur Herstellung enantiomerenangereicherter N-ungeschützter β-Aminosäuren durch enzymatische Hydrolyse eines Enantiomerengemisches von N-ungeschützten β-Aminosäurealkylestern oder β-Aminosäurearylestern mit einer Lipase unter der Maßgabe, dass kein entsprechender Methyl- oder Ethylester eingesetzt wird.

2. Verfahren nach Anspruch 2,
**dadurch gekennzeichnet, dass**
ein ensprechender n-Propyl-, i-Propyl, n-Butyl, sec-Butyl-, i-Butyl oder tert.-Butylester eingesetzt wird.

3. Verfahren nach einem oder mehreren der vorhergehenden Ansprüche,
**dadurch gekennzeichnet, dass**
der pH-Wert der Reaktion zwischen 4 und 10 liegt.

4. Verfahren nach einem oder mehreren der vorhergehenden Ansprüche,
**dadurch gekennzeichnet, dass**
die Temperatur bei der Reaktion zwischen -15 und +100°C liegt.

5. Verfahren nach einem oder mehreren der vorhergehenden Ansprüche,
**dadurch gekennzeichnet, dass**
man die Lipase PS aus *Pseudomonas cepacia* einsetzt.

6. Verfahren nach einem oder mehreren der vorhergehenden Ansprüche,
**dadurch gekennzeichnet, dass**
man die Reaktion in einem Enzym-Membran-Reaktor durchführt.

7. Verfahren nach einem oder mehreren der vorhergehenden Ansprüche,
**dadurch gekennzeichnet, dass**
man die Hydrolyse in wässrigem Medium durchführt, dem wasserlösliche organische Lösungsmittel zugemischt sein können.

8. Verfahren nach einem oder mehreren der vorhergehenden Ansprüche, **dadurch gekennzeichnet, dass** man die Hydrolyse in wässrigem Medium durchführt, dem wasserlösliche organische Lösungsmittel zugemischt sein können.

## Claims

1. Process for preparing enantiomer-enriched N-unprotected β-amino acids by enzymatic hydrolysis of an enantiomeric mixture of N-unprotected β-amino acid alkyl esters or β-amino acid aryl esters with a lipase provided that no corresponding methyl or ethyl ester is employed.

2. Process according to Claim 1, **characterized in that** a corresponding n-propyl, i-propyl, n-butyl, sec-butyl, i-butyl or tert-butyl ester is employed.

3. Process according to one or more of the preceding claims, **characterized in that** the pH of reaction is between 4 and 10.

4. Process according to one or more of the preceding claims, **characterized in that** the temperature during the reaction is between -15 and +100°C.

5. Process according to one or more of the preceding claims, **characterized in that** the lipase PS from *Pseudomonas cepacia* is employed.

6. Process according to one or more of the preceding claims, **characterized in that** the reaction is carried out in an enzyme membrane reactor.

7. Process according to one or more of the preceding claims, **characterized in that** the hydrolysis is carried out in aqueous medium to which watersoluble organic solvents may be admixed.

8. Process according to one or more of the preceding claims, **characterized in that** the hydrolysis is carried out in aqueous medium to which watersoluble organic solvents may be admixed.

## Revendications

1. Procédé de préparation d'acides β-aminés non N-protégés, enrichis en énantiomères, par hydrolyse enzymatique d'un mélange d'énantiomères d'esters alkyliques d'acides β-aminés ou d'esters aryliques d'acides β-aminés non N-protégés avec une lipase à condition qu'aucun ester méthylique ou éthylique correspondant ne soit utilisé.

2. Procédé selon la revendication 1, **caractérisé en ce qu'**un ester n-propylique, isopropylique, n-butylique, sec-butylique, isobutylique ou tert-butylique correspondant est utilisé.

3. Procédé selon l'une ou plusieurs des revendications précédentes, **caractérisé en ce que** le pH de la réaction est compris entre 4 et 10.

4. Procédé selon l'une ou plusieurs des revendications précédentes, **caractérisé en ce que** la température lors de la réaction est comprise entre -15 et +100°C.

5. Procédé selon l'une ou plusieurs des revendications précédentes, **caractérisé en ce que** l'on utilise la lipase PS de *Pseudomonas cepacia.*

6. Procédé selon l'une ou plusieurs des revendications précédentes, **caractérisé en ce que** la réaction est effectuée dans un réacteur à membrane enzymatique.

7. Procédé selon l'une ou plusieurs des revendications précédentes, **caractérisé en ce que** l'hydrolyse est effectuée dans un milieu aqueux avec lequel des solvants organiques hydrosolubles peuvent être mélangés.

8. Procédé selon l'une ou plusieurs des revendications précédentes, **caractérisé en ce que** l'hydrolyse est effectuée dans un milieu aqueux avec lequel des solvants organiques hydrosolubles peuvent être mélangés.
